(19) Europäisches Patentamt / European Patent Office / Office européen des brevets

(11) **EP 3 973 783 A1**

(12) **EUROPEAN PATENT APPLICATION**

(43) Date of publication:
**30.03.2022 Bulletin 2022/13**

(51) International Patent Classification (IPC):
***A23F 3/06*** *(2006.01)*      ***A23F 3/12*** *(2006.01)*

(21) Application number: **20198605.6**

(52) Cooperative Patent Classification (CPC):
**A23F 3/06; A23F 3/12**

(22) Date of filing: **28.09.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Unilever IP Holdings B.V.
3013 AL Rotterdam (NL)**

(72) Inventor: **The designation of the inventor has not
yet been filed**

(74) Representative: **Mewburn Ellis LLP
Aurora Building
Counterslip
Bristol BS1 6BX (GB)**

(54) **PREBIOTIC COMPOSITION**

(57)      The present invention relates a composition for use as a prebiotic, the composition comprising black leaf tea particles wherein the black leaf tea particles have a particle size of less than 250 μm.

EP 3 973 783 A1

**Description**

**Field of the invention**

The present invention relates to a composition for use as a prebiotic. In particular the present invention relates to a composition comprising black leaf tea particles for use as a prebiotic.

**Background of the invention**

Gastrointestinal health is a major concern of consumers in both developed and developing countries from the point of view of daily gastrointestinal comfort and also resistance to or prevention of acute and chronic diseases such as infectious diarrhoea and inflammatory bowel diseases. There is a demand for products that can be consumed on a daily basis that can provide such benefits.

Currently available strategies to address these concerns include probiotic products wherein liquids (typically dairy-based drinks) containing one or a number of strains of beneficial bacteria are consumed to derive a benefit such as improved gut health and/or improved natural defences. This approach is limited since it requires the products to be chilled (to maintain viability of the bacteria that provide the benefit), and questions remain over its efficacy since the bacteria must pass through the harsh acidic environment of the stomach and reach the colon in sufficient numbers to provide the intended effects. Furthermore, such considerations mean that probiotic products are typically expensive for the consumer and require a complex supply chain to effectively deliver them.

An alternative strategy is the use of prebiotics wherein food components are given that have a beneficial health effect through their selective metabolism in the intestinal tract. Typical targets for such an approach are bifidobacteria and lactobacilli (i.e. common probiotic species) since they are generally regarded as safe, are commonly found in breast fed infants in high numbers, alter the intestinal environment through the production of short chain fatty acids meaning that pathogens cannot colonise, produce antimicrobial components that target pathogens, may improve the immune status of the host and may reduce inflammation of the colon associated with poor modern diets.

Currently available prebiotics include fructans such as inulin and fructo-oligosaccharides and these have been shown to significantly increase bifidobacteria in humans.

Prebiotics do not suffer from questions over their viability in the way probiotics do and they can be formulated into a number of product formats without loss of viability. However, in certain aspects, current prebiotics are difficult to formulate into common foodstuffs owing to their impact on product taste (fructans can be slightly sweet) and structure (they can change mouthfeel). Furthermore, products are often required to be formulated with higher than the effective amount of prebiotic owing to the fact that fructans can suffer degradation during pasteurisation, baking, sterilisation and similar processes. These considerations mean that prebiotic-containing processed food products remain expensive and thus limited to only a small number of customers because of price.

Thus a demand exists from consumers for an affordable solution wherein benefits can be provided in the arena of gut health, resistance to infectious diseases and/or prevention of chronic gastrointestinal diseases.

Tea has been consumed in one form or another for over 4000 years and is popular in both developed and developing countries. Tea is popular for a number of reasons: it is generally regarded as healthy, it can be a safe alternative to untreated water and is associated with benefits such as relaxation, mental alertness and has a widely acceptable taste. The low price of tea also means that it can be consumed by consumers in all socio-economic groups.

Several documents suggest that consumption of black tea and/or components thereof can have positive effects on the health of the consumer. For example, US 2008/119545 A and US 2008/075795 A (both in the name of C. HENSLEY & S. PYO) disclose methods and compositions for preventing and treating Avian Influenza utilizing an effective quantity of an ingredient having a composition selected from the group consisting of theaflavin, theaflavin-3,3'-digallate, theaflavin-3-monogallate, theaflavin-3 gallate, theaflavin-3'-gallate, thearubigin, gallic acid, tannic acid, (-)-epigallocatechin gallate (EGCG), (-) epigallocatechin (EGC), (+)-epicatechin (EC), (-)-gallocatechin gallate (GCG), and catechin.

It has also been suggested that black tea and/or components thereof can have positive effects on gut function and/or health. For example, K. Jafari et al (Medical Hypotheses, 2006, 67(2), p.419) discloses that black tea extract and its major polyphenolic pigment may ameliorate the gastrointestinal disorder in irritable bowel syndrome; L. Chaudhuri et al (Life Sciences, 2000, 66(9), pp. 847-854) discloses that a hot water extract of black tea significantly accelerated the gastrointestinal transit (GIT) in vivo in mice; and S.E. Besra et al. (Phytotherapy Research, 2003, 17, pp. 380-384) discloses antidiarrhoeal activity of hot water extract of black tea.

Compositions comprising black tea components in combination with conventional prebiotics are also known. For example, WO 2007/056432 A (PERQUE INC) discloses compositions comprising one or more prebiotics (e.g., one or more dietary fibers) in combination with selenium compounds, flavonoids and/or flavonols, and phosphatides; and US 2008/085349 A (Z.Y. CHEN) discloses a beverage comprising tea components and a non-digestible health sweetener (VitaSugar® IMO).

It has also been recognised that black tea and/or components thereof can have a positive influence on gut microflora.

US 5,071,653 (ITOEN LTD) discloses substantially flavorless extracts from the leaves of C. sinensis which promote the growth of bifidobacteria. The compositions are provided by extracting water or ethanol-soluble solids from C. sinensis leaves with a polar organic solvent that is immiscible with water. There is no evidence in US 5,071,653 that the extracts disclosed therein have a true prebiotic effect (i.e. that promotion of the growth of bifidobacteria is in preference to promotion of the growth of pathogenic bacteria such as clostridia). Furthermore, the extracts disclosed therein are substantially flavourless and thus are presumably devoid of the flavonoids (such as thearubigin) which give black tea its distinctive taste.

WO 2004/056205 A (UNILEVER) discloses the use of a cooked food product comprising black tea leaves, an extract of black tea or a mixture thereof, to maintain or improve microflora balance and/or to treat or prevent diarrhoea in a subject consuming the composition and wherein the black tea leaves or the extract of black tea is/are present in an unbound state.

More recently, the prebiotic effect of the flavonoid components of black tea (and in particular, the therubigins has been reported.

WO2010/133404 A (UNILEVER) discloses use of a composition comprising black tea flavonoids as a prebiotic and/or for the treatment or prevention of conditions associated with poor gut health or low immunity. The flavonoids comprise thearubigin in an amount of at least 82% by weight of the tea flavonoids.

We have now identified that black leaf tea that has been divided into very fine particles has improved prebiotic effects compared with black tea infusion and even where the infusion comprise at least the same amount of black tea flavonoids. Thus the black leaf tea particles may be expected to be especially effective at delivering the health benefits associated with consumption of prebiotics, such as treatment or prevention of a gastrointestinal condition, and/or treatment or prevention of a condition associated with sub-optimal immunity.

**Tests and Definitions**

PREBIOTIC

As used herein, the term "prebiotic" refers to a substance consumed orally by an individual to beneficially affect that individual by selectively stimulating the growth and/or activity of one or more of a limited number of bacteria in the colon of the individual. The preferred prebiotics are those which selectively stimulate the growth and/or activity of bifidobacteria and/or lactic acid bacteria. Even more preferred are those which stimulate the growth and/or activity of bifidobacteria and/or lactic acid bacteria in preference to pathogenic bacteria such as clostridia.

Prebiotic effects in the gut can be evaluated on the basis of the growth of health promoting bacteria such as lactobacilli and bifidobacteria, the decrease in intestinal pathogens and/or the increase or decrease in production of health-related bacterial metabolites. The latter include for instance short-chain fatty acids (acetate, propionate and butyrate), which are generally believed to be positive for colonic health, while ammonia and branched short-chain fatty acids are regarded as risk factors for colon carcinogenesis.

TEA

As used herein, the term "tea" refers to material from the leaves and/or stem of Camellia sinensis var. sinensis and/or Camellia sinensis var. assamica. "Black tea" refers to tea wherein the leaves and/or stem have been subjected to a so-called "fermentation" step wherein they are oxidised by certain endogenous enzymes. This oxidation may even be supplemented by the action of exogenous enzymes such as oxidases, laccases and peroxidases.

"Leaf tea" for the purposes of this invention means tea leaves and/or stem in an uninfused form, and that has been dried to a moisture content of less than 30% by weight, and usually has a water content in the range 1 to 10% by weight.

PARTICLE SIZE

When referring to leaf tea particle size, this refers to the size of the tea particles capable of passing through (in respect of a maximum particle size) or retained on (in respect of a minimum particle size) a screen with apertures of the referenced aperture size. For example, leaf tea particles with a particle size of less than 250 $\mu$m can pass through a screen having a Tyler mesh size of 60 mesh; and leaf tea particles with a particle size of at least 90 $\mu$m are retained on a screen having a Tyler mesh size of 170 mesh.

FLAVONOIDS, THEARUBIGIN AND CATECHINS

Definitions and analytical methods for determining flavonoids and thearubigin can be found in Christiane Lakenbrink, Svenja Lapczynski, Beate Maiwald, and Ulrich H. Engelhardt. J. Agric. Food Chem., 2000, 48 (7), 2848-2852. In brief, total flavonoids and thearubigin in a composition can be calculated by the following relations (1) and (2):

$$\text{Total flavonoids(wt\%)} = TP - (GA + TG + CQA) \qquad (1),$$

$$\text{Thearubigin (wt\%)} = \text{Total flavonoids} - (C + TF + FOG + FAG) \qquad (2),$$

wherein $TP$ is the percentage of total phenolics by weight of the composition; $GA$ is the percentage of gallic acid by weight of the composition; $TG$ is the percentage of theogallin by weight of the composition; $CQA$ is the percentage of cholorogenic acids by weight of the composition; $C$ is the percentage of catechins by weight of the composition; $TF$ is the percentage of theaflavins by weight of the composition; $FOG$ is the percentage of flavonol glycosides (expressed as aglycons) by weight of the composition; and $FAG$ is the percentage of flavone glycosides (expressed as aglycons) by weight of the composition.

**Summary of the invention**

In a first aspect, the present invention provides use of a composition comprising black leaf tea particles as a prebiotic wherein the black leaf tea particles have a particle size of less than 250 $\mu$m.

Surprisingly we have found that black leaf tea particles having a particle size of less than 250 $\mu$m have improved prebiotic effects compared with black tea infusion solids. Thus the invention may also be said to relate to:

- a composition comprising black leaf tea particles for use as a prebiotic wherein the black leaf tea particles have a particle size of less than 250 $\mu$m;
- a method of producing a prebiotic effect in an individual, the method comprising administering to the individual a composition comprising an effective amount of black leaf tea particles wherein the black leaf tea particles have a particle size of less than 250 $\mu$m;
- a composition comprising black leaf tea particles for use as a medicament wherein the black leaf tea particles have a particle size of less than 250 $\mu$m;
- a composition comprising black leaf tea particles for use as a medicament in the treatment or prevention of a gastrointestinal condition, wherein the black leaf tea particles have a particle size of less than 250 $\mu$m;
- a composition comprising black leaf tea particles for use as a medicament in the treatment or prevention of a condition associated with sub-optimal immunity, wherein the black leaf tea particles have a particle size of less than 250 $\mu$m;
- a composition comprising black leaf tea particles for use as a medicament for the removal or alleviation of visceral pain, wherein the black leaf tea particles have a particle size of less than 250 $\mu$m;
- use of a composition comprising black leaf tea particles having a particle size of less than 250 $\mu$m in the manufacture of a medicament for the treatment or prevention of a gastrointestinal condition;
- use of a composition comprising black leaf tea particles having a particle size of less than 250 $\mu$m in the manufacture of a medicament for the treatment or prevention of a condition associated with sub-optimal immunity;
- use of a composition comprising black leaf tea particles having a particle size of less than 250 $\mu$m in the manufacture of a medicament for the removal or alleviation of visceral pain.

In a further aspect, the present invention provides a beverage comprising black leaf tea particles dispersed in an aqueous liquid, wherein the black leaf tea particles have a particle size of less than 250 $\mu$m.

The beverage is suitable for application in the uses listed above. Furthermore the very fine particle size (i.e. particle size of less than 250 $\mu$m) means that the leaf tea particles can remain suspended in the aqueous liquid for an extended time and/or do not produce an unpleasant grainy texture when the beverage is drunk.

The invention also provides a method of manufacturing the beverage, the method comprising the steps of:

(a) providing the aqueous liquid; and
(b) dispersing the black leaf tea particles in the aqueous liquid.

In a still further aspect the present invention provides a packaged beverage precursor suitable for use in the method, the precursor comprising a composition comprising black leaf tea particles packaged in a container, wherein the amount of the composition packaged in the container is from 0.5 to 500g and wherein the black leaf tea particles have a particle size of less than 250 μm.

Except in the Examples, all numbers in this description indicating amounts of material, time periods, length scales, conditions of reaction, physical properties of materials and/or use may optionally be understood as modified by the word "about".

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example, a method of use) such disclosure is also to be considered to apply to any other aspect of the invention (for example, a composition) *mutatis mutandis.*

## Detailed description

PREBIOTIC AND IMMUNOMODULATORY USES AND METHODS

In one aspect of the present invention, a composition comprising black leaf tea particles is administered to an individual in order to produce a prebiotic effect in the individual. The resulting prebiotic effect, i.e. the selective stimulation of the growth and/or activity of one or more of a limited number of bacteria may beneficially affect the health of the individual in one or more ways.

For example, administration of the composition may be used to treat or prevent a condition associated with sub-optimal immunity. The condition may, for example, be a viral infection, chronic inflammation and/or allergy. The condition associated with sub-optimal immunity is preferably a viral infection selected from influenza, common cold and/or a coronavirus.

Additionally or alternatively, administration of the composition may treat or prevent a gastrointestinal condition. The gastrointestinal condition is preferably selected from inflammatory bowel disease, irritable bowel syndrome, infectious diarrhoea, environmental enteropathy or a combination thereof.

The composition may additionally or alternatively be administered for the removal or alleviation of visceral pain.

The composition is administered orally.

The composition comprises an effective amount of the black leaf tea particles. In particular the amount of black leaf tea particles administered to the individual on a daily basis should be enough to produce a noticeable prebiotic effect in the gut microflora of the individual. Thus it is preferred that the composition is administered to the individual in an amount to provide at least 0.5 g black leaf tea particles per day, more preferably at least 1.0 g black leaf tea particles per day and most preferably at least 1.5 g black leaf tea particles per day. Large amounts of black leaf tea particles need not be administered in order to achieve the prebiotic effect. Thus it is preferred that the composition is administered to the individual in an amount to provide at most 20 g black leaf tea fparticles per day, more preferably at most 15 g black leaf tea particles per day, more preferably still at most 10 g black leaf tea particles per day and most preferably at most 6 g black leaf tea particles per day.

BLACK LEAF TEA PARTICLES

The black leaf tea particles for use in the compositions, uses, beverages, beverage precursors and methods of the present invention are have a particle size of less than 250 μm, preferably less than 200 μm, more preferably less than 150 μm and most preferably less than 100 μm. Although there is no particular limitation on the lower end of the particle size range, the black leaf tea particles preferably have a particle size of greater than 5 μm, more preferably greater than 10 μm, still more preferably greater than 20 μm.

Where tea particles are present in the composition but outside of the specified particle size, then it is preferred that at least 85% by weight of black leaf tea particles has this particles size, more preferably at least 90% by weight, still more preferably at least 95% by weight, and most preferably from 99% to 100% by weight. The weight percentage of particles having a particular particle size can be determined by sorting into fractions according to particle size (e.g. by sieving) and then weighing the fractions. When determining particle size, the leaf tea particles are in a dried format and have a moisture content of less than 30 wt.% (typically 1 to 10 wt.%).

Leaf tea contains material having a range of particle sizes as a result of the normal manufacturing process. Indeed, leaf tea is typically sorted into various grades (e.g. whole leaf, brokens, fannings and dusts) prior to being sold at auction. One way of sorting tea is according to particle size. For example, the leaf tea may be passed through a series of vibrating screens where the various grades are retained and collected. Leaf tea particles having a particle size of less than 250 $\mu$m could be separated in this manner. For example, the leaf tea can be sieved through a screen having a Tyler mesh size of 60 mesh.

Although it is possible to obtain black leaf tea particles from commercially available black leaf tea by sieving (as described above), this is not very efficient as only a small proportion of the leaf tea will be of the appropriate size. Thus, the particles are preferably provided by a step of grinding black leaf tea to obtain black leaf tea particles in order to increase the proportion of the leaf tea having the appropriate particle size. For example, the leaf tea can be ground using impact mills, hammer mills, ball mills, jet mills, cone mills, roll mills, stone mills, centrifugal mills and the like. The ground leaf tea can optionally be passed through one or a series of screens as described above in order to select the fraction of black leaf tea particles having the specified particle size.

As the tea particles of the present invention are in an uninfused form, they will comprise significant amounts of flavonoids. Preferably the black leaf tea particles comprise at least 2% flavonoids by weight of the black leaf tea particles, more preferably at least 4%, and most preferably from 6 to 20%.

The black tea flavonoids for use in the compositions, uses, beverages, beverage precursors and methods of the present invention are preferably enriched in thearubigin. For example, the black tea flavonoids may comprise thearubigin an an amount of at least 70% by weight of the flavonoids. More preferably the black tea flavonoids comprise at least 82% by weight of the tea flavonoids, most preferably in an amount of at least 84% by weight of the tea flavonoids.

Typically the black tea flavonoids will comprise non-thearubigin components such as theaflavins and/or catechins. Thus the amount of thearubigin in the black tea flavonoids may be less than 99.99% by weight of the black tea flavonoids, or even less than 98% by weight of the black tea flavonoids. It is especially preferred that amount of catechins in the black tea flavonoids is limited, as catechins appear to reduce the prebiotic effect of thearubigins. Thus it is preferred that the flavonoids comprise catechins in an amount of less than 12% by weight of the flavonoids, more preferably in an amount of less than 10% by weight of the flavonoids, more preferably still less than 8%, and most preferably from 0.01 to 6%. Additionally or alternatively, the black tea flavonoids may comprise less than 3% theaflavins by weight of the tea flavonoids as theaflavins have little or no prebiotic effect. More preferably the black tea flavonoids comprise less than 1.5% theaflavins by weight of the black tea flavonoids, more preferably still less than 0.5% and most preferably from 0.0001 to 0.1%.

BEVERAGE AND BEVERAGE PRECURSOR

In a preferred aspect of the present invention, the composition is administered to the individual in the form of an edible product. The edible product may be a food. For example, the product may be a margarine, low fat spread, confectionery product (such as chocolate), ice cream, yoghurt, dressing, mayonnaise, sauce, bakery product, shortening, soup or cheese. However, the very small particle size of the black leaf tea particles make them especially suitable for suspending in an aqueous medium to form a beverage. Thus it is especially preferred that the edible product is a beverage, most preferably a tea-based beverage.

The beverage comprising black leaf tea particles dispersed in an aqueous liquid in a sufficient amount to provide that a prebiotic effective dose of the particles can be achieved without having to consume a large number of beverages in a single day. Thus the beverage preferably comprises the black leaf tea particles in a mass of at least 0.5 g, more preferably greater than 1.0 g, and most preferably at least 1.5 g. However, the product need not comprise large amounts of black leaf tea particles. In particular it is preferred that the mass of black leaf tea particles in the product is at most 20 g, more preferably at most 15 g, more preferably still at most 10 g and most preferably at most 5 g.

The mass of the beverage should not be so large that an excessive amount of beverage is required to be consumed on a daily basis. Thus the beverage has a mass of less than 500 g, preferably less than 450 g, more preferably from 50 to 350 g.

Because black tea particles can influence the colour and/or texture of a beverage, it is preferable to employ the particles in a limited concentration range. In particular it is preferred that the concentration of black leaf tea particles is from 0.15 to 10% by weight of the beverage, more preferably from 0.2 to 4%.

The beverage may be manufactured in any suitable way, however in a preferred embodiment the beverage is manufactured using a method comprising:

(a) providing the aqueous liquid; and
(b) dispersing the black leaf tea particles in the aqueous liquid.

Preferably the aqueous liquid and the tea particles are combined in a weight ratio of at least 5 parts aqueous to 1 part black leaf tea particles, more preferably in a weight ratio of 10:1, more preferably still 50:1 and most preferably 100:1 to 1000:1.

This method allows for separately manipulating the properties of the aqueous liquid and black leaf tea particles before combination. Furthermore, the higher proportion of aqueous liquid than black leaf tea particles allows for minimisation of the impact of the particles on texture and/or colour of the liquid.

The liquid is an aqueous liquid i.e. contains greater than 50% water by weight of the liquid. In some embodiments the aqueous liquid may be in the form of an oil-in-water or a water-in-oil emulsion, preferably oil-in-water.

Preferred aqueous liquids are water, milk and vegetable milk analogues (such as almond milk, soya milk, oat milk etc.). Most preferably the aqueous liquid is water.

Prior to being dispersed in the aqueous liquid, the black leaf tea particles are preferably packaged. Thus the beverage precursor of the invention preferably comprises a composition comprising black leaf tea particles packaged in a container.

The mass of the precursor composition packaged in the container is preferably sufficient to prepare at least one beverage. For example the amount may be at least 0.5 g, more preferably at least 1 g and most preferably at least 1.5 g. In some embodiments the package may contain multiple servings and so the amount of composition in the container may be, for example up to 500 g, more preferably up to 250 g. In an especially preferred embodiment, however, the amount packaged is the amount needed for a single serving. Thus it is preferred that the amount is at most 5 g, more preferably at most 4 g and most preferably at least 3.5 g.

As the prebiotic effect is produced by whole leaf tea particles it is preferred that the container in which the precursor composition is packaged is suitable for dosing the composition into an aqueous liquid rather than containing them during infusion (as would be the case, for example with a tea bag). Thus it is especially preferred that the container is a water-resistant (as opposed to water permeable) container such as a sachet, bottle, tub, carton or pouch.

The present invention will now be described, by way of example only, with reference to the following Examples.

**Examples**

Tea Materials

Powdered soluble black tea solids (referred to hereafter as "Black Infusion Powder") was prepared as follows: Kenyan BOP (Broken Orange Pekoe) grade black tea was added to 90°C water (1:10 tea leaf to water weight ratio) and allowed to infuse for 20 minutes. The tea leaves were subsequently removed, and the resulting infusion freeze-dried.

Micronized black leaf tea (referred to hereafter as "Black Micronized Leaf Powder") was prepared as follows: Kenyan BOP (Broken Orange Pekoe) grade black tea was milled using an ultra-centrifugal mill (model ZM 100 from Retsch GmbH & Co. KG). The mill was operated with a load control of up to 10, a speed of 14000 rpm and an inner filter/sieve size of 80 $\mu$m (ring filter). This starting BOP material had a particle size of about 1.4 mm, whilst the Black Micronized Leaf Powder had a particle size of less than 80 $\mu$m.

Measurement of Prebiotic Effects

Overall microbial fermentation (pH and gas production) and microbial metabolic activity in terms of production of saccharolytic (short-chain fatty acids and lactate) and proteolytic end-products (ammonium) were studied in-vitro for three healthy adult donors. Effects of the tea materials on microbial community structure were assessed through 16S-targeted Illumina sequencing.

*Short-term colonic incubations*

The short-term screening assay consisted of a colonic incubation of a single dose of the tea materials under conditions representative for the proximal colon region of an adult human, using a representative bacterial inoculum.

At the start of the short-term colonic incubation, the Black Infusion Powder was added in a concentration of 1% (w/v) and the Black Micronized Leaf Powder at 1.5% (w/v) to sugar-depleted nutritional medium containing basal nutrients present in the colon (e.g. host-derived glycans such as mucin). A blank, containing only the sugar-depleted nutritional background medium was included also, allowing to assess the background activity of the bacterial community. The reason for using less Black Infusion Powder was to account for the relatively higher content of polyphenols (including thearubigin) in that material compared with the Black Micronized Leaf Powder.

As a source of the colonic microbiota a fecal inoculum of three donors was added (healthy adult humans not drinking tea). Incubations were performed for 48h at 37°C, under shaking (90 rpm) and anaerobic conditions. The incubations were performed in fully independent reactors with sufficiently high volume to not only allow a robust microbial fermentation, but also to allow collection of multiple samples overtime. Sample collection enables assessment of metabolite production and allows to understand the complex microbial interactions that are taking place. Each condition was performed in triplicate to account for biological variation, resulting in 27 independent incubations (2 treatments and 1 blank, tested in triplicate in three donors).

*Endpoints of short-term incubations*

- Overall microbial fermentation

pH: the degree of acidification during the experiment is a measure for the intensity of bacterial metabolism of the test product. The pH of the incubations was determined at 0, 6, 24 and 48h after starting the incubation, thus giving a rough indication on the speed of fermentation of the different tea materials.

Gas production: the colon incubations were performed in closed incubation systems. This allowed to evaluate the accumulation of gasses in the headspace, which can be measured with a pressure meter. Gas production is a measure of microbial activity, and thus of the speed of fermentation of the tea materials. Gas production during the incubations was determined at 0, 6, 24 and 48h after starting the incubation.

- Changes in microbial activity

The following analyses were used to compare the different tea materials:
Short chain fatty acids (SCFA): This is an assessment of the microbial carbohydrate metabolism (acetate, propionate and butyrate) or protein metabolism (branched SCFA) and can be compared to typical fermentation patterns for normal GI microbiota. Samples for SCFA analysis were analyzed after 0, 6, 24 and 48h of incubation.

Lactate: The human intestine harbors both lactate-producing and lactate-utilizing bacteria. Lactate is produced by lactic acid bacteria and decreases the pH of the environment, thereby also acting as an antimicrobial agent. Protonated lactic acid can penetrate the microbial cell after which it dissociates and releases protons within the cell, resulting in acidification and microbial cell death. It can also be rapidly converted into especially butyrate by other microorganisms. Samples for lactate analysis were analyzed after 0, 6, 24 and 48h of incubation.

Ammonium: This is a product of proteolytic degradation, which results in the production of potentially toxic or carcinogenic compounds such as p-cresol and p-phenol. It can be used as an indirect marker for low substrate availability. Since it is only produced towards the end of the incubation, it was measured after 24 and 48h, not after 6h of incubation

- Changes in microbial community composition (Illumina)

Two techniques were combined according to Vandeputte et al. [(2017) "Quantitative microbiome profiling links gut community variation to microbial load". Nature: 551, 507-511] to map the community shifts induced by the different tea materials in large detail: 16S-targeted Illumina sequencing and flow cytometry.

16S-targeted Illumina sequencing: This is a PCR-based method by which microbial sequences are amplified until saturation, thus providing proportional abundances of different taxa at different phylogenetic levels (microbial phylum, family and OTU level). Illumina was performed at the start and after 24h and 48h of incubation. The methodology involved primers that span 2 hypervariable regions (V3-V4) of the 16S rRNA gene. Using a pair-end sequencing approach, sequencing of 2x250bp results in 424 bp amplicons. Such fragments are taxonomically more informative than smaller fragments.

Quantification of total bacterial cells by flow cytometry: Samples that were analyzed with Illumina sequencing were also analyzed with flow cytometry to determine the number of total bacterial cells, thus allowing to convert the proportional values obtained with Illumina into absolute quantities. Samples were analyzed on a BD Facs verse. The samples were run using the high flow rate. Bacterial cells were separated from medium debris and signal noise by applying a threshold level of 200 on the SYTO channel. Proper parent and daughter gates were set to determine all populations.

Results

*Microbial metabolic activity*

pH: Besides the pH decrease that was inherent to product addition due to the slightly acidic nature of the tea materials, both materials resulted in a significantly stronger pH decrease than the blank during the first 6h of incubation ($p < 0.05$) in each of the donors. This indicates that product fermentation started at an early stage of the incubation. The strongest initial pH decreases were obtained with the Black Infusion Powder in donor A; both materials resulted in similar pH decreases (i.e. not significantly different from one another) in donors B and C. Tea material fermentation consistently resulted in a further pH decrease during the 6-24h timeframe in each of the three donors (significantly stronger than the blank ($p < 0.05$)), suggesting that the tea materials were not yet depleted after 6h. While the pH remained relatively stable in the blank incubations during the final 24h of the incubation, significant further pH decreases were observed for both tea materials in donors A and B, and for the Black Micronized Leaf Powder in donor C (p<0.05). Overall, the strongest pH decreases were consistently observed for the Black Micronized Leaf Powder.

Gas production: Both materials stimulated gas production during the first 6h in all donors, as observed from significantly higher gas pressures in the treatment incubations than the blank (p<0.05). These observations confirm that product fermentation started at an early stage of the incubation. Tea material fermentation consistently stimulated gas production during both the 6-24h and 24-48h timeframes in each of the three donors (significantly higher gas pressures in the tea incubations than the blank (p<0.05)), suggesting that the products were not yet depleted after 6h. Overall, the highest gas pressures after 48h were consistently obtained with the Black Micronized Leaf Powder.

Total SCFA: Both tea materials yielded significantly higher total SCFA levels than the blank in each donor (p<0.05). In donor C, both materials stimulated SCFA production already during the first 6h of the incubation. After 6h, similar SCFA concentrations were obtained for both materials in donors A and B. By the end of the 48h incubation, the Black Micronized Leaf Powder had consistently yielded the highest SCFA concentrations.

Acetate: Fermentation of both tea materials started already during the first 6h of the incubation. At the end of the incubation, both materials had yielded significantly higher acetate concentrations than the blank (p<0.05). The highest acetate concentrations were consistently obtained for the Black Micronized Leaf Powder.

Propionate: Both materials stimulated the production of propionate during the first 6h of the incubation. By the end of the incubation, both materials had yielded significantly higher propionate concentrations than the blank (p<0.05). Interindividual differences were observed in the sense that the highest propionate concentrations in donors A and B were obtained for the Black Micronized Leaf Powder, while for donor C the highest propionate concentrations resulted from fermentation of the Black Infusion Powder.

Butyrate: Both materials significantly increased butyrate levels compared to the blank in each of the donors tested (p<0.05), indicating that fermentation of tea material strongly contributed to butyrate production. Butyrate concentrations were low after 6h of incubation, which was expected, considering that butyrate is a secondary metabolite requiring primary production of acetate and/or lactate. Therefore, it is typically produced during later stages of the incubation, in this case between 6-24h. As for propionate, interindividual differences were observed, in the sense that the highest butyrate concentrations at the end of the incubation were obtained for Black Micronized Leaf Powder in donors A and C, but for Black Infusion Powder in donor B.

Branched SCFA: Overall, production of branched SCFA mainly occurred during the 6-48h timeframe. Both materials yielded branched SCFA concentrations significantly lower than the blank in donors A and B (p<0.05). In donor C, branched SCFA concentrations were similar to the blank, although very low branched SCFA concentrations were obtained for blank and treatments in this specific donor.

Ammonium: Production of ammonium mainly occurred during the first 24h of the incubation. In donors A and C, both materials resulted in similar ammonium concentrations as the blank. In donor B, however, both materials were found to significantly lower production of ammonium relative to the blank (p<0.05).

Lactate: Both materials significantly increased lactate levels compared to the blank incubation during the first 6h (p<0.05). Lactate accumulation implies that during the first 6h the lactate production rate was higher than the lactate consumption rate. The highest lactate concentrations after 6h were consistently obtained with Black Micronized Leaf Powder. Overall, lactate produced during the first 6h was efficiently consumed after 24h in all incubations. This is indicative of efficient lactate conversion. Lactate consumption was consistently highest for Black Micronized Leaf Powder, which is related with the higher lactate concentrations that were measured after 6h.

*Microbial Community shifts*

Shifts at the total count and phylum levels are presented in Table 1 for 24h incubation and Table 2 for 48 hr incubation. Values are given as cells/ml.

For all three donors the total numbers of bacterial cells were most strongly increased for the Black Micronized Leaf Powder.

TABLE 1

| Donor | Phylum | Blank | Black Infusion Powder | Black Micronized Leaf Powder |
|---|---|---|---|---|
| A | Proteobacteria | 6.63E+08 | 5.23E+08 | 9.54E+08 |
| | Firmicutes | 8.22E+08 | 1.89E+09 | 1.45E+09 |
| | Bacteroidetes | 7.27E+08 | 2.34E+08 | 9.83E+08 |
| | Actinobacteria | 2.30E+08 | 2.92E+08 | 4.74E+08 |
| | **Total** | **2.44E+09** | **2.94E+09** | **3.86E+09** |
| B | Proteobacteria | 8.28E+08 | 4.01 E+08 | 7.38E+08 |
| | Firmicutes | 8.00E+08 | 1.10E+09 | 1.82E+09 |
| | Bacteroidetes | 2.61 E+08 | 4.77E+07 | 3.75E+08 |
| | Actinobacteria | 5.12E+08 | 5.28E+08 | 1.15E+09 |
| | **Total** | **2.40E+09** | **2.08E+09** | **4.08E+09** |
| C | Proteobacteria | 4.81 E+08 | 1.51 E+08 | 1.88E+08 |
| | Firmicutes | 8.92E+08 | 1.55E+09 | 2.64E+09 |
| | Bacteroidetes | 7.07E+08 | 4.45E+08 | 7.37E+08 |
| | Actinobacteria | 2.55E+08 | 2.83E+08 | 9.43E+08 |
| | **Total** | **2.34E+09** | **2.43E+09** | **4.51 E+09** |

TABLE 2

| Donor | Phylum | Blank | Black Infusion Powder | Black Micronized Leaf Powder |
|---|---|---|---|---|
| A | Proteobacteria | 8.30E+08 | 6.58E+08 | 1.32E+09 |
| | Firmicutes | 7.21 E+08 | 1.79E+09 | 1.61 E+09 |
| | Bacteroidetes | 8.04E+08 | 3.08E+08 | 1.28E+09 |
| | Actinobacteria | 3.22E+08 | 2.78E+08 | 4.82E+08 |
| | **Total** | **2.68E+09** | **3.03E+09** | **4.69E+09** |
| B | Proteobacteria | 9.80E+08 | 5.06E+08 | 9.93E+08 |
| | Firmicutes | 6.62E+08 | 1.38E+09 | 1.98E+09 |
| | Bacteroidetes | 2.89E+08 | 7.58E+07 | 3.78E+08 |
| | Actinobacteria | 4.94E+08 | 5.41 E+08 | 1.10E+09 |
| | **Total** | **2.43E+09** | **2.50E+09** | **4.45E+09** |
| C | Proteobacteria | 6.07E+08 | 3.62E+08 | 3.88E+08 |
| | Firmicutes | 8.22E+08 | 1.66E+09 | 2.39E+09 |
| | Bacteroidetes | 6.12E+08 | 6.66E+08 | 1.16E+09 |
| | Actinobacteria | 2.39E+08 | 3.05E+08 | 1.00E+09 |
| | **Total** | **2.28E+09** | **2.99E+09** | **4.94E+09** |

Analysis of the fecal inocula showed that, although the three donors were found to share similarities in terms of the most abundant bacterial phyla that occupied their gut microbiota, significant differences were observed at the bacterial family and operational taxonomic unit (OUT) level. For this reason, it is useful to describe treatment effects at these levels separately for each donor.

Donor A: Both materials resulted in the enrichment of Bifidobacteriaceae between 0-24h. Higher levels than the blank were maintained during the 24-48h timeframe and were attributed to OTU3 and OTU14, identified as closely related to *Bifidobacterium adolescentis/faecale* and *B. longum* subsp. *longum/null,* respectively. Each of the materials stimulated at least one member of the Bacteroidaceae family. Overall, the Black Micronized Leaf Powder resulted in an immediate stimulatory effect (i.e. between 0-24h), while the Black Infusion Powder tended to stimulate Bacteroidaceae during later stages of the incubation (24-48h). Further, while the Black Micronized Leaf Powder increased overall abundance of this family strongly, the Black Infusion Powder rather decreased overall abundances of Bacteroidaceae. The following observations were made: Black Infusion Powder stimulated OTU4 (*Bacteroides vulgatus*) between 24-48h. Black Micronized Leaf Powder stimulated OTU4 (*Bacteroides vulgatus*), OTU17 (*Bacteroides eggerthii*) and OTU32 (*Bacteroides massiliensis*) between 0-24h and these stimulatory effects were maintained until the end of the incubation. OTU18 (*Bacteroides ovatus*) was stimulated by the Black Micronized Leaf Powder during a later stage of the incubation, i.e. between 24-48h. Both materials had an immediate stimulatory effect on Enterococcaceae, which lasted until the end of the incubation. Black Micronized Leaf Powder stimulated Erysipelotrichaceae throughout the 48h incubation period and this stimulatory effect was attributed to OTU24, identified as closely related to *Holdemanella.* Each material stimulated different members of the Lachnospiraceae family: Black Infusion Powder stimulated OTU12 (*Fusicatenibacter saccharivorans*) and OTU21 (Butyrate-producing bacterium/*Eubacterium rectale*) throughout the 48h incubation period and OTU72 (unresolved identity) during the first 24h of the incubation. Black Micronized Leaf Powder stimulated OTU12 (*Fusicatenibacter saccharivorans*), OTU21 (Butyrateproducing *bacterium/Eubacterium rectale*), OTU26 (*Blautia faecis*) and OTU73 (Member of Clostridium cluster XIVb) throughout the 48h incubation period, and OTU72 (unresolved identity) between 0-24h. Both materials stimulated Ruminococcaceae throughout the 48h incubation period, with stimulatory effects dedicated to OTU9 and OTU10, both identified as closely related to *Faecalibacterium prausnitzii,* and OTU29, with unresolved identity. Black Micronized Leaf Powder stimulated members of the Burkholderiaceae family throughout the 48h incubation period and Enterobacteriaceae during a later stage of the incubation (24-48h). Stimulatory effects on Enterobacteriaceae were consistently attributed to OTU1, identified as closely related to *Escherichia coli/Shigella sonnei.*

Donor B: Black Micronized Leaf Powder resulted in the enrichment of Bifidobacteriaceae throughout the 48h incubation. Black Infusion Powder stimulated Bifidobacteriaceae during a later stage of the incubation (24-48h). Stimulatory effects were attributed to OTU3, closely related to *Bifidobacterium adolescentis/faecale.* Black Micronized Leaf Powder additionally stimulated OTU27, identified as closely related to Bifidobacterium bifidum. Black Micronized Leaf Powder stimulated Atopobiaceae and mildly stimulated Coriobacteriaceae throughout the 48h incubation. The Black Micronized Leaf Powder also had a stimulatory effect on Eggerthellaceae throughout the 48h incubation period which was attributed to OTU8, identified as closely related to *Senegalimassilia.* Black Micronized Leaf Powder also stimulated members of the Bacteroidaceae family, more specifically OTU4, identified as closely related to *Bacteroides vulgatus,* and had a mild stimulatory effect on members of the Barnesiellaceae family during a later stage of the incubation (24-48h). The Black Micronized Leaf Powder resulted in the enrichment of members of the Prevotellaceae family already during the first 24h of the incubation and the enrichment was maintained until the end of the incubation and was attributed to OTU60 (*Prevotella* sp.). The Black Infusion Powder stimulated Prevotellaceae during a later stage of the incubation (between 24-48h). Black Infusion Powder had an immediate stimulatory effect on Enterococcaceae, which lasted until the end of the incubation. The stimulatory effect on Enterococcaceae by Black Micronized Leaf Powder occurred during a later stage of the fermentation (24-48h). Stimulatory effects on Enterococcaceae were attributed to OTU2, closely related to *Enterococcus durans/hirae/faecium/azikeevi/villorum.* Black Micronized Leaf Powder stimulated Erysipelotrichaceae between 24-48h of incubation and the stimulatory effects were attributed to OTU24, identified as closely related to *Holdemanella.* Both materials stimulated different members of the Lachnospiraceae family, where the following observations were made: Both stimulated OTU7 (*Dorea longicatena*), OTU30 (*Blautia obeum*), OTU43 (*Coprococcus eutactus*/Butyrate-producing bacterium) and OTU45 (*Blautia obeum*). Black Infusion Powder co-enriched OTU25 (*Ruminococcus faecis/R. torques*) throughout the 48h incubation period. Black Micronized Leaf Powder co-stimulated OTU12 (*Fusicatenibacter saccharivorans*), OTU25 (*Ruminococcus faecis/R. torques*), OTU46 (*Roseburia*) and OTU48 (*Eubacterium eligens*) throughout the 48h incubation. Black Micronized Leaf Powder moderately stimulated Lactobacillaceae. Each material stimulated different members of the Ruminococcaceae family: Both materials stimulated OTU9 and OTU10 between 0-24h, both closely related to *Faecalibacterium prausnitzii,* with significantly higher levels than the blank maintained during the 24-48h timeframe in the incubations with Black Micronized Leaf Powder. Black Infusion Powder co-stimulated OTU11 (*Gemmiger formicilis*) between 0-24h. and significantly higher levels than the blank were maintained during the 24-48h timeframe. Black Micronized Leaf Powder co-stimulated OTU11 (*Gemmiger formicilis*) and OTU37 (*Flavonifractor*) between 0-24h with significantly higher levels than the blank maintained during the 24-48h timeframe and OTU28 (*Faecalibacterium prausnitzii*) co-enriched between 24-48h. Both materials stimulated members of the Streptococcaceae family, resulting in significantly higher abundances than the blank throughout the incubation. The Black Micronized Leaf Powder stimulated members of the Burkholderiaceae family throughout the 48h incubation period. The Black Infusion Powder stimulated Burkholderiaceae during a later stage of the incubation (24-48h).

Donor C: Both materials resulted in the enrichment of members of the Bifidobacteriaceae family between 0-24h, more specifically OTU3 (*Bifidobacterium adolescentis/faecale*), with higher levels than the blank maintained during the 24-48h timeframe in the incubations with the Black Micronized Leaf Powder. OTU14 (*B.longum* subsp. *longum*/*null*) was co-enriched between 24-48h in the incubations with Black Micronized Leaf Powder. Both materials stimulated Coriobacteriaceae between 0-24h with higher levels than the blank were maintained until the end of the incubation. Both materials stimulated specific members of the Bacteroidaceae family, while overall the Black Micronized Leaf Powder stimulated members of the Bacteroidaceae family most profoundly. Both materials stimulated OTU4 (*Bacteroides vulgatus*) between 0-24h with higher levels than the blank were maintained until the end of the incubation. Both materials coenriched OTU5 at some point of the incubation. Black Micronized Leaf Powder formulations stimulated members of the Tannerellaceae family throughout the incubation. Clostridiaceae were moderately stimulated by the Black Micronized Leaf Powder between 0-24h. Black Infusion Powder stimulated Erysipelotrichaceae between 0-24h. Black Micronized Leaf Powder stimulated Erysipelotrichaceae at a later stage of the incubation (between 24-48h). Both materials stimulated different members of the Lachnospiraceae family, where the following observations were made: Both stimulated OTU34 (*Anaerostipes*) and OTU41 (*Clostridium glycyrrhizinilyticum*) over the 48h incubation period. Black Micronized Leaf Powder co-enriched OTU49 (*Clostridium citroniae*/*clostridioforme*) and OTU54 (Butyrate-producing bacterium) between 0-24h. Both materials stimulated members of the Ruminococcaceae family and this was attributed to a stimulatory effect on OTU9 (*Faecalibacterium prausnitzii*), OTU11 (*Gemmiger formicilis*) and OTU57 (*Butyricicoccus*). Both materials stimulated Streptococcaceae and this was mainly attributed to OTU6, identified as closely related to *Streptococcus salivarius* subs. *null*. Black Micronized Leaf Powder stimulated *Veillonellaceae* between 24-48h and this was attributed to OTU13, identified as closely related to *Veillonella dispar*. Black Micronized Leaf Powder stimulated Burkholderiaceae during a later stage of the incubation (24-48h) with the stimulatory effect dedicated to OTU44, identified as closely related to *Sutterella wadsworthensis*.

Conclusions

The short-term colonic incubations illustrated that both tea materials were efficiently fermented by the gut microbiota of the three human adult donors under investigation, resulting in elevated SCFA (acetate, propionate and butyrate) and lactate levels, with minor interindividual differences being observed in terms of metabolite production.
The Black Micronized Leaf Powder resulted in stronger pH decreases, higher gas production and stronger total SCFA production than the Black Infusion Powder. Acetate concentrations were also consistently higher for the Black Micronized Leaf Powder and it was the Black Micronized Leaf Powder that most profoundly stimulated lactate production.
Production of acetate and lactate suggests the action of lactic acid bacteria, while production of propionate suggests that also Bacteroidetes spp., typically capable of degrading complex molecules, may have been involved in substrate breakdown. Illumina data confirmed this hypothesis, in the sense that both materials consistently enriched members of the Bifidobacteriaceae family in each of the three investigated donors.
For all three donors the total numbers of bacterial cells were most strongly increased for the Black Micronized Leaf Powder.
Thus it can be concluded that the Black Micronized Leaf Powder had a significantly greater prebiotic effect than the Black Infusion Powder.

**Claims**

1. Use of a composition comprising black leaf tea particles as a prebiotic wherein the black leaf tea particles have a particle size of less than 250 $\mu$m.

2. A composition comprising black leaf tea particles for use as a medicament wherein the black leaf tea particles have a particle size of less than 250 $\mu$m.

3. A composition comprising black leaf tea particles for use as a medicament in the treatment or prevention of a condition associated with sub-optimal immunity, wherein the black leaf tea particles have a particle size of less than 250 $\mu$m.

4. Composition as claimed in claim 3, wherein the condition is a viral infection, chronic inflammation and/or allergy.

5. Composition or use as claimed in claim 4, wherein the viral infection is infection with influenza, common cold and/or a coronavirus.

6. A composition comprising black leaf tea particles for use as a medicament in the treatment or prevention of a gastrointestinal condition, wherein the black leaf tea particles have a particle size of less than 250 $\mu$m.

7. Composition as claimed in claim 6, wherein the condition is selected from inflammatory bowel disease, irritable bowel syndrome, environmental enteropathy, and infectious diarrhoea.

8. A composition comprising black leaf tea particles for use as a medicament for the removal or alleviation of visceral pain, wherein the black leaf tea particles have a particle size of less than 250 μm.

9. Use as claimed in claim 1 or composition as claimed in any one of claims 2 to 8, wherein the black leaf tea particles comprise at least 2% flavonoids by weight of the black leaf tea particles.

10. Use or composition as claimed in claim 9 wherein the flavonoids comprise thearubigin in an amount of at least 70% by weight of the tea flavonoids, preferably in an amount of at least 82% by weight of the tea flavonoids.

11. Use or composition as claimed in any one of the preceding claims wherein the composition is administered to an individual in an amount of at least 0.5 g black leaf tea particles per day.

12. A beverage comprising black leaf tea particles dispersed in an aqueous liquid, wherein the black leaf tea particles have a particle size of less than 250 μm.

13. The beverage as claimed in claim 12 wherein the beverage has a mass of less than 500 g and comprises the black leaf tea particles in an amount of at least 0.5 g.

14. A method of manufacturing a beverage as claimed in claim 12 or 13, the method comprising:

(a) providing the aqueous liquid; and
(b) dispersing the black leaf tea particles in the aqueous liquid.

15. A packaged beverage precursor comprising a composition comprising black leaf tea particles packaged in a container, wherein the amount of the composition packaged in the container is from 0.5 to 500 g and wherein the black leaf tea particles have a particle size of less than 250 μm.

Europäisches
Patentamt

European
Patent Office

Office européen
des brevets

# EUROPEAN SEARCH REPORT

Application Number

EP 20 19 8605

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XIAO WEIHUA ET AL: "A method for producing superfine black tea powder with enhanced infusion and dispersion property", FOOD CHEMISTRY, vol. 214, 1 January 2017 (2017-01-01), pages 242-247, XP055781226, NL ISSN: 0308-8146, DOI: 10.1016/j.foodchem.2016.07.096 | 12-15 | INV. A23F3/06 A23F3/12 |
| Y | * the whole document * | 1-15 | |
| Y | CONRAD ASTILL ET AL: "Factors Affecting the Caffeine and Polyphenol Contents of Black and Green Tea Infusions", J. AGRIC. FOOD CHEM., vol. 49, no. 11, 1 January 2001 (2001-01-01), pages 5340-5347, XP055513800, DOI: 10.1021/jf010759+ * the whole document * | 1-15 | |
| Y,D | WO 2010/133404 A1 (UNILEVER PLC [GB]; UNILEVER NV [NL] ET AL.) 25 November 2010 (2010-11-25) * claim 1 * | 1-15 | TECHNICAL FIELDS SEARCHED (IPC) A23F |
| Y | LEE H C ET AL: "Effect of tea phenolics and their aromatic fecal bacterial metabolites on intestinal microbiota", RESEARCH IN MICROBIOLOGY, ELSEVIER AMSTERDAM, NL, vol. 157, no. 9, 1 November 2006 (2006-11-01), pages 876-884, XP027954838, ISSN: 0923-2508 [retrieved on 2006-11-01] * the whole document * | 1-15 | |

-/--

The present search report has been drawn up for all claims

| Place of search | Date of completion of the search | Examiner |
|---|---|---|
| The Hague | 2 March 2021 | Merel-Rausch, Eva |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 20 19 8605

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2004/056205 A1 (UNILEVER NV [NL]; UNILEVER PLC [GB] ET AL.) 8 July 2004 (2004-07-08) * the whole document * | 1-15 | |
| Y | BESRA S E ET AL: "ANDIDIARRHOEAL ACTIVITY OF HOT WATER EXTRACT OF BLACK TEA (CAMELLIA SINENSIS)", PHYSIOTHERAPY RESEARCH, JOHN WILEY & SONS LTD. CHICHESTER, GB, vol. 14, no. 7, 1 April 2003 (2003-04-01), pages 380-384, XP009011900, ISSN: 0951-418X, DOI: 10.1002/PTR.1171 * the whole document * | 1-15 | |
| Y | JAFARI ET AL: "Black tea extract and its major polyphenolic pigment may ameliorate the gastrointestinal disorder in irritable bowel syndrome", MEDICAL HYPOTHESES, EDEN PRESS, PENRITH, US, vol. 67, no. 2, 1 January 2006 (2006-01-01), page 419, XP005476704, ISSN: 0306-9877, DOI: 10.1016/J.MEHY.2006.02.037 * the whole document * | 1-15 | |
| Y,D | US 2008/119545 A1 (HENSLEY CHARLES [US] ET AL) 22 May 2008 (2008-05-22) * claim 1 * | 1-15 | TECHNICAL FIELDS SEARCHED (IPC) |

The present search report has been drawn up for all claims

| Place of search | Date of completion of the search | Examiner |
|---|---|---|
| The Hague | 2 March 2021 | Merel-Rausch, Eva |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 19 8605

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-2021

| Patent document cited in search report | | Publication date | Patent family member(s) | | Publication date |
|---|---|---|---|---|---|
| WO 2010133404 | A1 | 25-11-2010 | CA | 2762665 A1 | 25-11-2010 |
| | | | CN | 102427824 A | 25-04-2012 |
| | | | EA | 201171421 A1 | 29-06-2012 |
| | | | EP | 2432485 A1 | 28-03-2012 |
| | | | PL | 2432485 T3 | 31-01-2014 |
| | | | US | 2010297058 A1 | 25-11-2010 |
| | | | WO | 2010133404 A1 | 25-11-2010 |
| | | | ZA | 201107768 B | 27-12-2012 |
| WO 2004056205 | A1 | 08-07-2004 | AP | 2039 A | 07-09-2009 |
| | | | AR | 042548 A1 | 22-06-2005 |
| | | | AU | 2003294731 A1 | 14-07-2004 |
| | | | BR | 0316135 A | 27-09-2005 |
| | | | CL | 2003002700 A1 | 08-04-2005 |
| | | | MX | PA05006434 A | 19-08-2005 |
| | | | TR | 200502229 T2 | 21-11-2006 |
| | | | WO | 2004056205 A1 | 08-07-2004 |
| US 2008119545 | A1 | 22-05-2008 | US | 2008119545 A1 | 22-05-2008 |
| | | | US | 2011257258 A1 | 20-10-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008119545 A
- US 2008075795 A
- WO 2007056432 A
- US 2008085349 A
- US 5071653 A
- WO 2004056205 A
- WO 2010133404 A

**Non-patent literature cited in the description**

- **K. JAFARI.** *Medical Hypotheses,* 2006, vol. 67 (2), 419
- **L. CHAUDHURI et al.** *Life Sciences,* 2000, vol. 66 (9), 847-854
- **S.E. BESRA et al.** *Phytotherapy Research,* 2003, vol. 17, 380-384
- **CHRISTIANE LAKENBRINK ; SVENJA LAPCZYNSKI ; BEATE MAIWALD ; ULRICH H. ENGELHARDT.** *J. Agric. Food Chem.,* 2000, vol. 48 (7), 2848-2852
- **VANDEPUTTE et al.** Quantitative microbiome profiling links gut community variation to microbial load. *Nature,* 2017, vol. 551, 507-511